Europäisches Patentamt

European Patent Office

Office européen des brevets

(11) Publication number: **0 334 141**
A1

## EUROPEAN PATENT APPLICATION

(21) Application number: 89104354.9

(51) Int. Cl.4: **A61N 1/42**

(22) Date of filing: 11.03.89

(30) Priority: 22.03.88 US 171837

(43) Date of publication of application:
27.09.89 Bulletin 89/39

(84) Designated Contracting States:
CH DE FR GB IT LI SE

(71) Applicant: Holcomb, Robert Ray
P.O. Box 779
Hamilton Alabama 35570(US)

(72) Inventor: Holcomb, Robert Ray
P.O. Box 779
Hamilton Alabama 35570(US)

(74) Representative: Meyers, Ernest et al
Office de Brevets FREYLINGER & ASSOCIES
B.P. 1 321, route d'Arlon
L-8001 Strassen(LU)

(54) Magnetic treatment device.

(57) A therapeutic permanent magnet device adapted for pain relieving placement on the bodies of living animals is provided. The device includes a plurality of magnet bodies (12, 14, 16, 18) having two positive and two negative magnetic poles and a containment body (20) for holding the magnet bodies in a fixed orientation. A method for therapeutically placing the permanent magnet device on the human body to relieve pain is also provided.

FIG. 1

## MAGNETIC TREATMENT DEVICE

The present invention relates to magnetic devices for therapeutic application to the human body, and more particularly to a quadrapolar static magnetic device for pain relieving placement on the human body.

Magnetic fields have been applied to the human body for various therapeutic purposes. For example, magnetic plasters for improving circulation are disclosed in United States Patent Number 4,489,711, magnetic fields for stimulation of bone growth are disclosed in U.S. Patent No. 4,105,017, and magnetic stimulation of nerve cells has been accomplished with devices such as the Cadwell Magneto-Electric Stimulator (MES-10) manufactured by Cadwell Laboratories, Inc. of Kennewick, Washington.

Recent studies indicate that static magnetic fields may be therapeutically applied in order to alter the behavior of nerve tissue. In a study, Hong, Harmon & Yu: Static Magnetic Field Influence on Rat Tail Nerve Function, 67 Arch. Phys. Med. Rehabil. 746-49 (1986), a static magnetic field having a density greater than .5 Tesla was found to alter nerve function when applied for a duration of at least 30 seconds. The Hong study postulates that an electromagnetic field may relieve pain by selectively increasing the excitability of large nerve fibers which, according to the gate control theory, may block the gate for pain. This study also suggests that static magnetic fields alter nerve func tion by stabilizing nerve cell membranes and the permeability of the membranes to certain ions.

Chronic pain sensations in the human body can be a result of improper nerve function, as when such pain is caused by inordinately excitable nerve cells or by nerve cells having leaky cell wall membranes. Chronic pain sensations may also be caused by damaged nerve cells, as for example nerve cells suffering from post-operative scarring or physically impinged nerve cells commonly associated with degenerative disc disease. Even when nerves function properly, chronic pain sensations are initiated through nerve cells. Thus, chronic pain relief should be obtainable by altering nerve cell function, as for example by stabilizing nerve cell wall membranes.

Unfortunately, many types of chronic pain cannot be successfully treated with conventional drug or physical therapies. Because chronic pain is often untreatable with conventional therapies, there is a need for alternative therapies that relieve pain by altering nerve function. Accordingly, it is an objet of the invention to provide a device that alters nerve behaviour in a manner that reduces chronic pain sensations. It is a further object of the invention to provide a method for applying a therapeutic permanent magnet device to the human body to relieve pain.

In accordance with the principles of the present invention, as embodied and as broadly described herein, a therapeutic permanent magnet device adapted for pain relieving placement on the bodies of living animals is provided. The device comprises a plurality of magnet bodies having at least two positive and two negative magnetic poles substantially in a single plane, the magnetic poles being oriented to define the four vertices of a quadrilateral shape, the two positive poles defining opposite diagonal vertices, and the two negative poles defining opposite diagonal vertices of the quadrilateral shape. Containment means are provided for holding the magnetic poles of the magnetic bodies in the quadrilateral orientation, and attachment means are provided for fixing the containment means on a living body. Preferably, the plurality of magnet bodies comprises four substantially identical cylindrical magnetic bodies, each having at least one magnetically charged face. It is further preferred that two of the cylindrical magnetic bodies have a positive magnetic pole on one face and two of the magnetic bodies have a negative magnetic pole on one face. and that the two positive and two negative magnetic poles on the magnetically charged faces on the four magnet bodies be in the quadrilateral orientation described above.

The accompanying drawings, which are incorporated in and constitute a part of this specification, illustrate presently preferred embodiments of the invention, and, together with the description, serve to explain the principles of the invention.

Fig. 1 is a prospective view of a magnetic treatment device according to one embodiment of the invention.

Fig. 2 is a plan view of the magnetic treatment device of Fig. 1.

Fig. 3 is a cross-sectional view taken along the line III-III of Fig. 1

Fig. 4 is a prospective view of one of the magnetic bodies of the magnetic treatment device of Fig. 1.

Fig. 5 is a plan view of a second embodiment of the magnetic treatment device of the invention.

Fig. 6 is a cross-sectional view taken along the linge VI-VI of Fig. 5.

Fig. 7 is a top view of the magnetic treatment device of Fig. 2, with dots added to designate surface magnetic field measurement locations.

Fig. 8 is a graph of magnetic filed measurements taken at the measurement locations designated in Fig. 7.

Figs. 9-15 are grids of magnetic filed measurements taken at various distances from the surface of the magnetic treatment device of the present invention.

Fig. 16 is a rear view of a human body showing the placement of a magnetic treatment device according to the invention.

Fig. 17 illustrates a method for applying magnetic treatment devices to cervical vertebrae.

Fig. 18 illustrates a method for applying magnetic treatment devices to lumbar and sacral vertebrae.

Fig. 19 illustrates a method for applying a magnetic treatment device to the knee.

Reference will now be made in detail to the presently preferred embodiments of the invention, examples of which are illustrated in the accompanying drawings. Throughout the drawings, like reference characters are used to designate like elements.

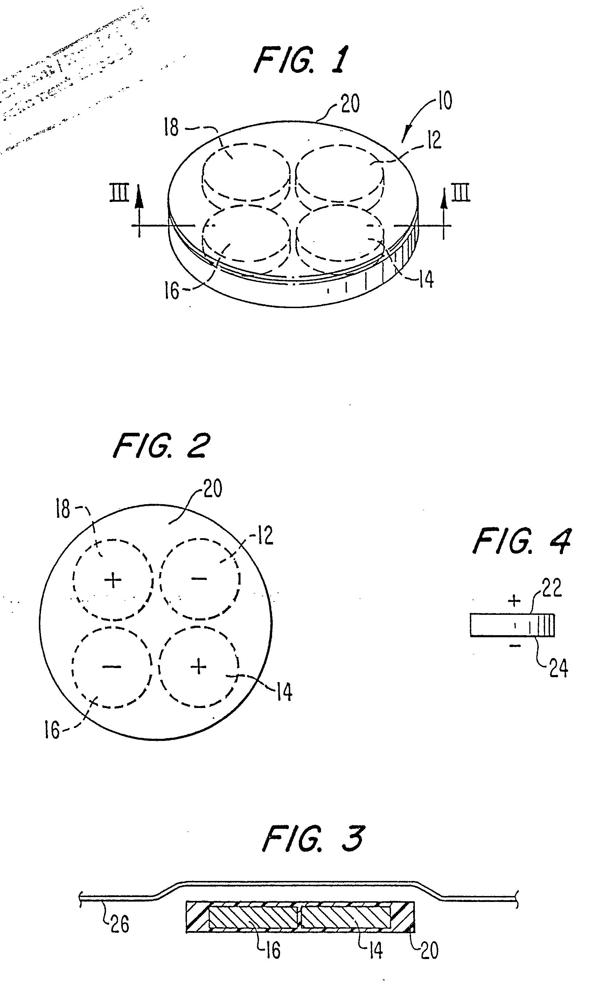

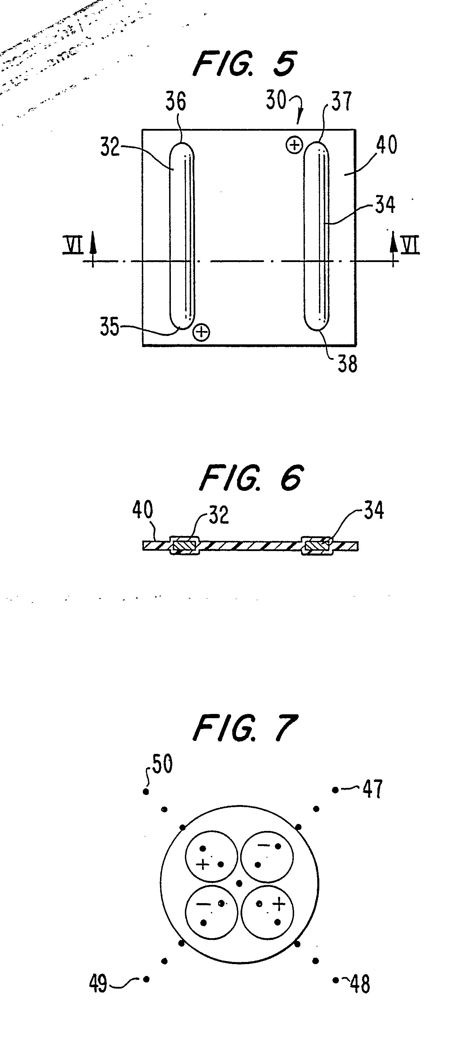

Two embodiments of a therapeutic permanent magnet device adapted for pain-relieving placement on the bodies of living animal bodies are shown in Figs. 1 and 5. In each embodiment of the invention, a plurality of magnet bodies are oriented and held in a containment body, which is adapted for pain-relieving placement on a living body.

The invention embodiment shown in Fig. 1 includes a permanent magnet device 10 having a plurality of magnet bodies 12, 14, 16 and 18 held in a housing 20. According to the invention, the plurality of magnet bodies have at least two positive and two negative magnetic poles substantially in a single plane, the magnetic poles being oriented to define four vertices of a quadrilateral shape, the two positive poles defining opposite diagonal vertices and the two negative poles defining opposite diagonal vertices of the quadrilateral shape.

As embodied herein, four permanent magnet bodies 12, 14, 16 and 18 are provided each having a generally cylindrical shape and two opposite faces. As shown in Fig. 4, one face 22 of each cylindrical body is positively charged, and the opposite face 24 of each cylindrical body is negatively charged. Preferably, a positive magnetic pole is located on face 22, while a negative magnetic pole is located on face 24. As shown in Figs. 1 and 2, magnetic bodies 12, 14, 16 and 18 are oriented to define the four vertices of a quadrilateral shape. The quadrilateral shape is preferably a square, as shown in Fig. 2, wherein the positively charged faces of magnetic bodies 14 and 18 and the negatively charged faces of magnetic bodies 12 and 16 are in single plane. The two positive poles on faces 14 and 18 define opposite diagonal vertices of the square shape, while the two negative poles on the faces of magnetic bodies 12 and 16 define opposite diagonal vertices of the square shape. Each of the four magnetic poles is magnetically attracted by the two oppositely charged poles and is magnetically repelled by the like charged pole.

Preferably, each of the magnet cylinders comprises a neodymium magnet, but other magnetic bodies, such as samarium-cobalt magnets and ferrite magnets, may also be advantageously utilized in the device. Each of the magnet bodies, preferably exhibits an energy product of at least 25 MG-Oe, and most preferable exhibits an energy product of 27 MG-Oe. Neodymium magnets manufactured by Delco Remy, a division of General Motors Corporation, exhibit the desired energy products and can be advantageously applied in the present invention. Preferably, the diameter of each magnet body is equal and is within the range of 6 mm to 13 mm. It is further preferred that each magnetic body have an equal thickness.

When cylindrical magnet bodies with opposite poles on opposite faces are utilized in the invention, opposite sides of magnetic device 10 will exhibit the same quadrapolar magnetic charge distribution. Whichever side of magnetic treatment device 10 is applied against the body of the treatment subject, the therapeutic effect will be the same.

According to the invention, containment means are provided for holding the magnetic poles of the magnetic bodies in the desired orientation. Containment body 20 may comprise a hypo-allergenic plastic in which four magnetic bodies 12, 14, 16 and 18 are embedded. Containment body 20 may alternatively comprise any thin woven sheath for holding magnetic bodies 12, 14, 16 and 18 in the desired quadrapolar orientation.

According to the invention, attachment means for affixing the containment means on a living body are provided. As shown in Fig. 3, the attachment means may comprise a hypo-allergenic adhesive pad 26, for holding containment body 20 in a desired placement position on the skin surface of a subject being treated. Treatment device 20 may be otherwise attached by a bandage, a plaster cast, glue, an elastic band, adhesive tape or any other equivalent attachment mechanism.

According to a second embodiment of the invention, as shown in Fig. 5, the therapeutic permanent magnet device of the invention may comprise two elongated permanent magnet bodies, 32 and 34, each having a positive pole and a negative pole. As shown in Fig. 6, elongated magnet bodies 32 and 34 are embedded in a containment body 40 comprised of a hypo-allergenic plastic. Positive pole 35 and negative pole 36 of a magnetic body 32 and positive pole 37 and negative pole 38 of magnetic body 34 are oriented to define the four vertices of a rectangular shape in which positive poles 35 and 37 define opposite diagonal vertices of the rectangular shape and negative poles 36 and 38 define opposite diagonal vertices of the rectangular shape. The embodiment of the invention shown in Fig. 5 and 6 is attached to a treatment subject in the same manner as disclosed for the invention embodiment shown in Figs. 1-3.

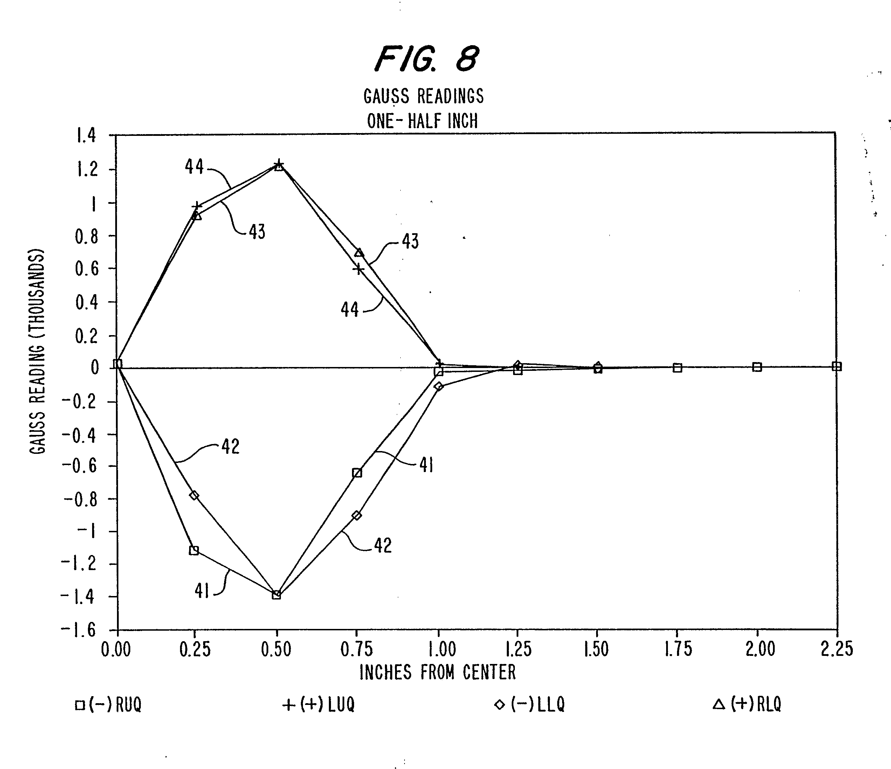

The magnetic field generated by a magnetic treatment device like that shown in Fig. 1 is quantified in the graphs and charts of Figs. 8-13. The measurements in Figs. 8-14 were made on a magnetic treatment device having the orientation shown in Fig. 1, in which each of the four magnetic bodies was a 12,7 mm diameter neodymium magnet, having an energy product of 27 MG-Oe. The magnetic field measurements were taken with a Gauss meter made by Applied Magnetic Laboratory Inc., and are recorded in Gauss.

Fig. 8 is a graph of magnetic field measurements taken along the surface of a quadrapolar magnetic treatment device like the one shown in Fig. 1 at measurement locations spaced .25 inch apart along diagonal lines as shown in Fig. 7. Graph line 41 in Fig. 8 corresponds to measurements taken along the diagonal 46-47 in Fig. 7; graph line 42 in Fig. 8 corresponds to measurements taken along the diagonal 46-49 in Fig. 7; graph line 43 corresponds to measurements taken along the diagonal 46-48 in Fig. 7; and graph line 44 corresponds to measurements taken along the diagonal 46-50 in Fig. 7. As shown in Fig. 8, magnetic field strength is negligible at the center of the magnetic treatment device, is greatest at one-half inch from the center of the magnetic treatment device in each of the four diagonal directions and decreases to almost nothing at 1.25 inches from the center of the magnetic treatment device. The quadrapolar arrangement of the present invention provides a magnetic field coning effect, wherein the magnetic field is negligible at the center of the treatment device and is greatest at approximately one-half inch from the center of the treatment device.

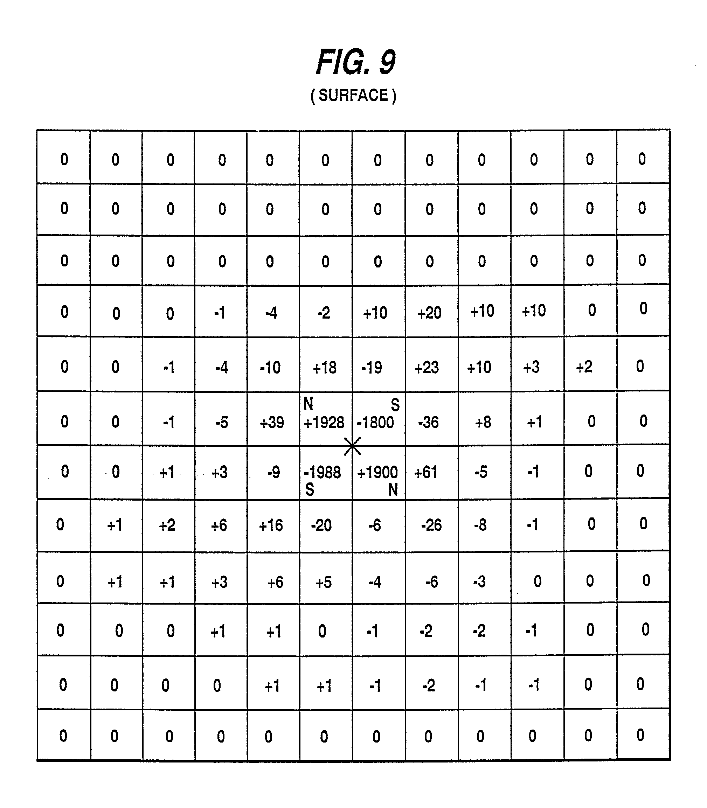

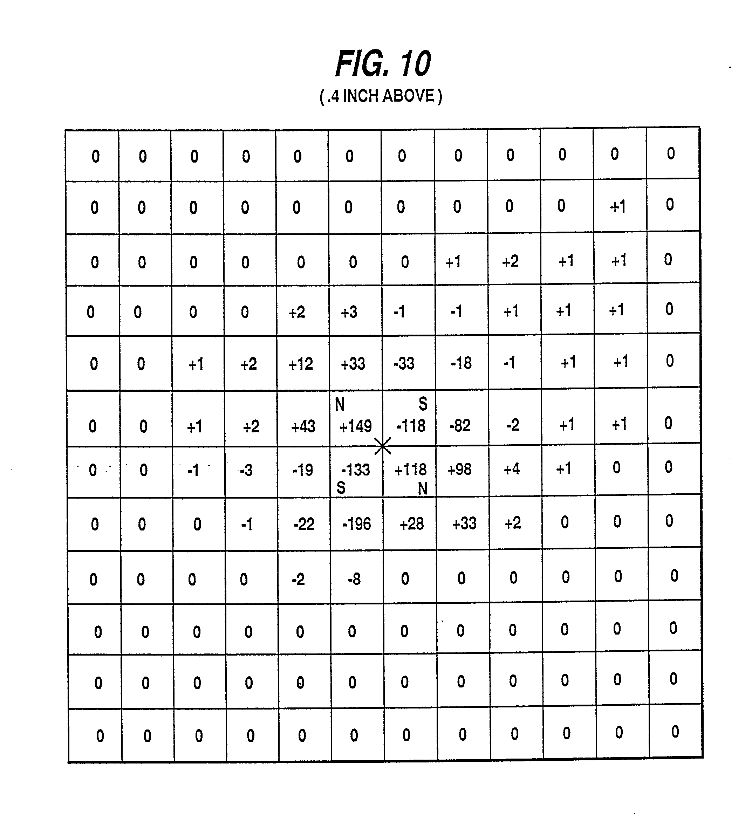

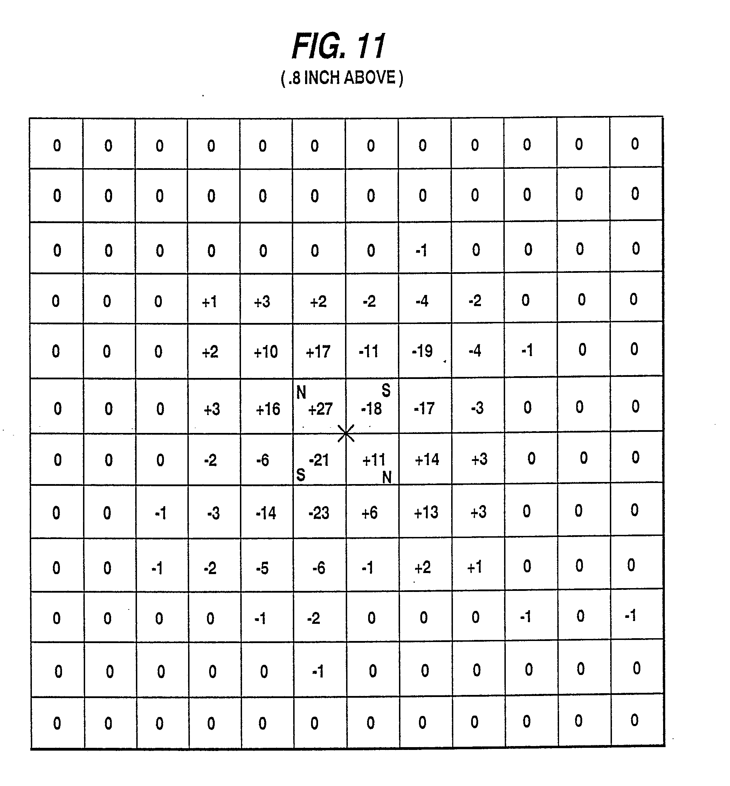

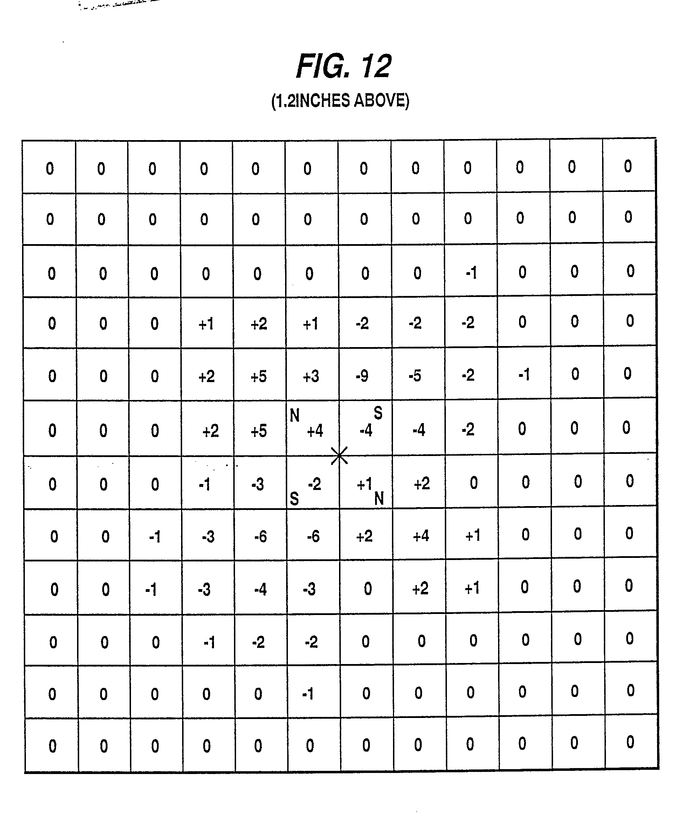

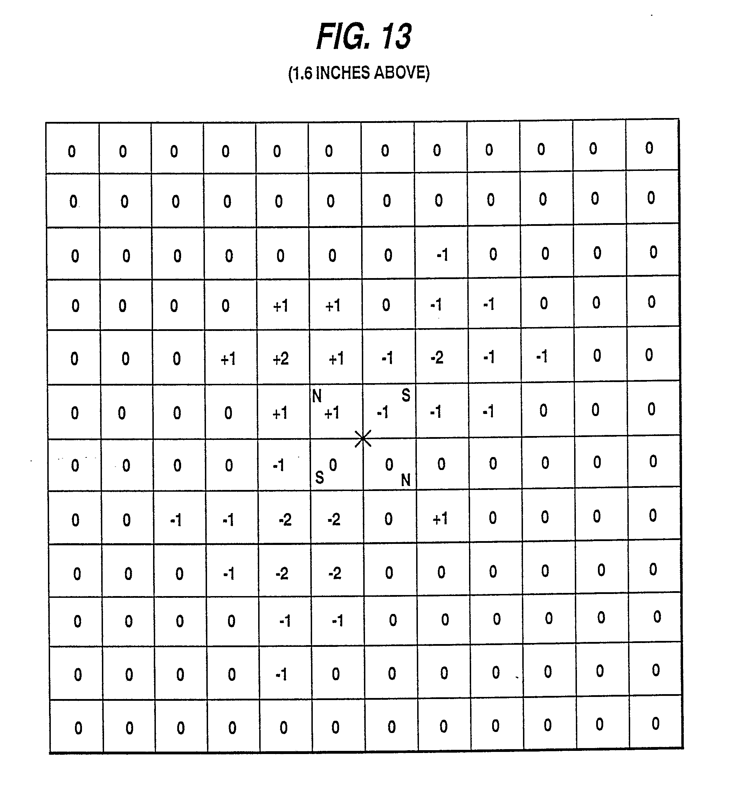

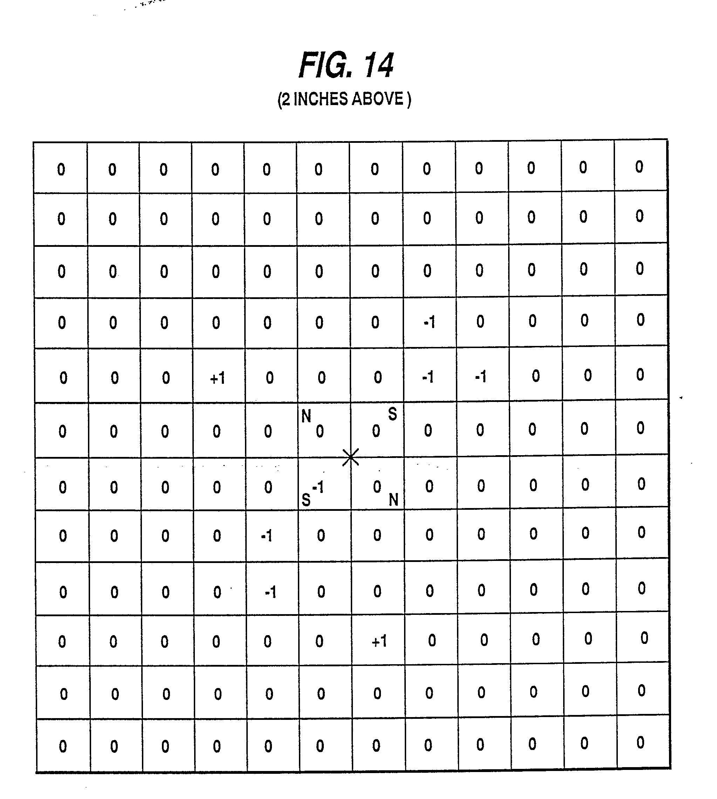

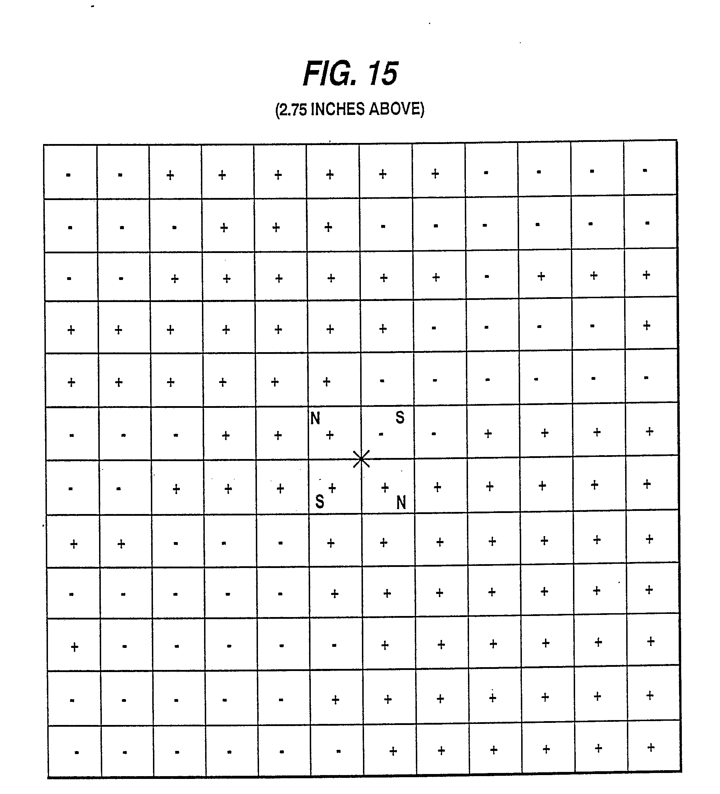

Figs. 9-15 show magnetic field measurements in Gauss taken at various distances from the surface of the magnetic treatment device described with regards to Figs. 7 and 8. The measurements shown in Figs. 9-15 were taken above the magnetic treatment device, and the location of the positive poles of the treatment device are designated are the letter "N" (north) and the negative poles on the face of the treatment device are designated by the letter "S" (south). The measurements were taken at the surface of the magnetic treatment device (Fig. 9), 10 mm above the surface (Fig. 10), 20 mm above the surface (Fig. 11), 30,5 mm above the surface (Fig. 12), 40,5 mm above the surface (Fig. 13), 50,8 mm above the surface (Fig. 14) and 70 mm above the surface (Fig. 15). The measurement grids of Figs. 9-15 are each 15,25 cm by 15,25 cm with individual grid squares of 12,7 mm by 12,7 mm. The Gauss meter was used to measure the magnetic field at the center of each grid square and the measurement was recorded on the corresponding grid square of Figs. 9-15.

As can be seen from this data, measurable magnetic flux extended out 50,8 mm from the surface of the treatment device. At 70 mm above the surface of the treatment device, the magnetic flux meter used in the test was not sensitive enough to measure specific flux levels, but the measuring instrument was able to distinguish between positive (north pole) and negative (south pole) flux readings as shown in Fig. 15. Thus, it can be seen than a quadrapolar magnetic field extends out as far as 70 mm from the surface of the magnetic treatment device of the present invention.

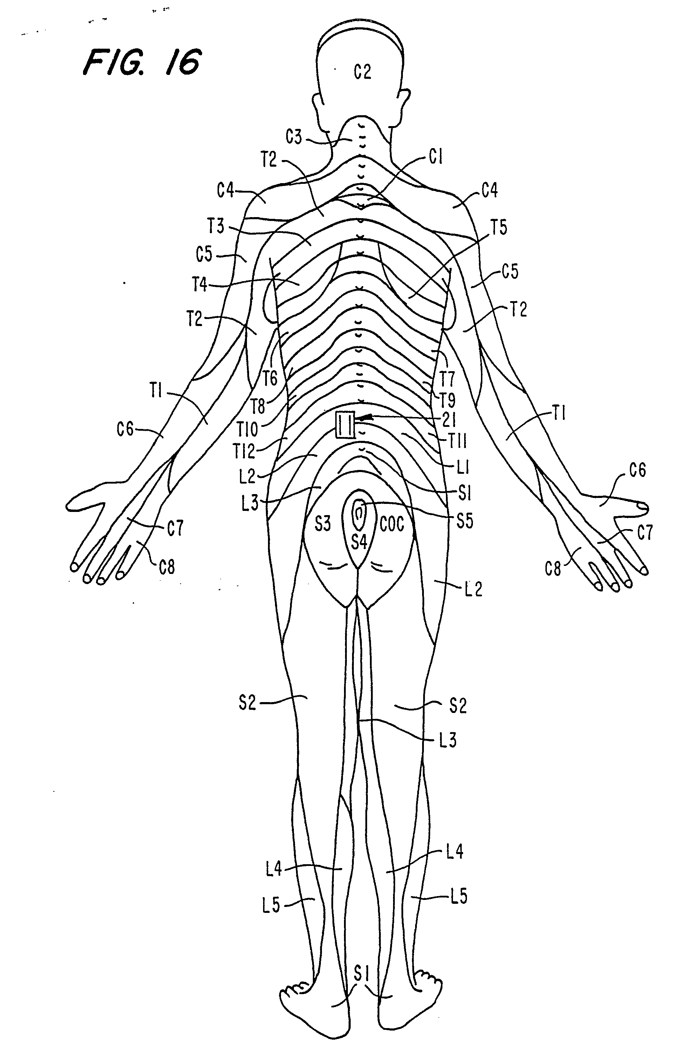

It has been found that the magnetic treatment device of the present invention may be advantageously applied to various parts of the human body to provide pain relief. The magnetic treatment device of the present invention has been advantageously applied to human test subjects for the relief of pain. When applied, the magnetic treatment device is attached to the skin over nerve bundles in various parts of the human body, particularly in the back area as shown with device 21 in Fig. 16. The magnetic treatment device has been found to be especially effective in the treatment of radicular pain, also known at root pain. One experiencing radicular pain senses pain sensations in the extremities, that are caused by improper nerve function in the spinal area. Such improper nerve function is frequently due to irritability and inflammation of the nerve root, a condition associated with degenerative disc disease, osteoarthritis, post-operative scarring, and arthritis. Placement of quadrapolar magnetic treatment devices according to the present invention over nerves producing pain sensations has been found in tests to significantly decrease radicular pain.

## EXAMPLE NO. 1

In a preliminary uncontrolled study on 396 human subjects, one or more pain syndromes being experienced by each study subject were treated with the magnetic treatment device of the present invention. Radicular pain syndromes associated with the cervical vertebrae were treated in 190 of the 396 test subjects. The cervical vertebrae comprise the top seven vertebrae in the spinal column and are located in the neck region. A plurality of magnetic treatment devices, as shown in Figs. 1-3, were applied to each test subject as follows.

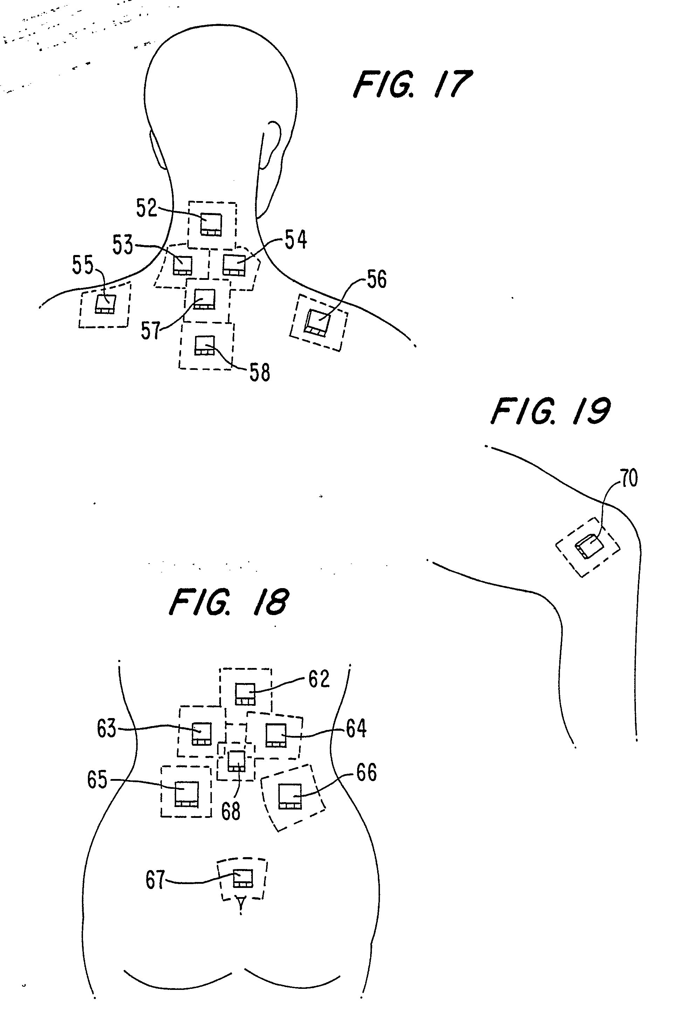

As shown in Fig. 17, seven magnetic treatment devices were applied from treatment of cervical radicular pain. Treatment device 52 was applied midline between vertebrae C-5 and C-6. Treatment devices 53 and 54 were applied on each side of spinous process C-6 and C-7. Treatment device 57 was applied midline over spinous process T-1 and T-2. Treatment device 58 was applied midline over spinous process T-3 and T-4. Treatment devices 55 and 56 were placed medial to each acro-clavicular joint. Each treatment device 52-58 was fixed in place with an orientation such that the nearest magnetic poles, facing the skin surface, of adjoining devices were charged opposite to each other. This overall placement technique was arrived at empirically.

In this preliminary study, the seven magnetic treatment devices applied as described above were left in place for a period as short as three hours to as long as several months. Each subject was asked to quantify the pain they were experiencing on a pain intensity scale of 0 to 10 where zero represented no pain and 10 represented intolerable pain. Each subject assigned a value to the pain he was experiencing associated with the cervical vertebrae both before and after the treatment. The percentage pain relief was then calculated. For example, if a subject assigned a pain value of 8 to his pain before treatment and a pain value of 4 to his pain after treatment, the percentage pain relief would equal 50% ((8-4)/8). The results on 190 cervical radicular pain syndromes were as follows.

| Pain Syndrome Category | Number of Syndromes Treated | Average % Relief of Pain Reported |
|---|---|---|
| Degenerative disc disease | 126 | 85.4 % |
| Osteoarthritis | 33 | 83.0 % |
| Post-operative scarring | 3 | 80.9 % |
| Arthritis | 23 | 86.9 % |

## EXAMPLE NO. 2

In the preliminary uncontrolled tests discussed in Example No. 1, run on 396 subjects who each reported one or more pain syndromes, pain associated with the lumbar and sacral vertebrae was also treated. Radicular pain associated with the lumbar and sacral vertebrae was treated in 315 of the test subjects. Six magnetic treatment devices, as shown in Fig. 1, were applied to each test subject reporting a lumbar or sacral radicular pain syndrome. As shown in Fig. 18, one treatment device 67 was attached over the proximal one-third of the cocyx. Treatment devices 65 and 66 were placed over the dimple of the venus bilaterally. Treatment devices 63 and 64 were attached one-half inch toward midline bilaterally approximately three inches above the dimples of the venus over L-3. One treatment device 62 was attached one-half inch toward midline bilaterally approximately three inches above L-3 (over spinous process L-1). Subsequent to this test, ist has been found advantageous to place an additional device 68 midline between L-3 and L-4. Each treatment device 62-67 was fixed in place with an orientation such that the nearest magnetic poles, facing the skin surface, of adjoining devices were charged opposite to each other. This overall placement technique was arrived at empirically. The attached devices were left in place for a period as short as three hours to a period as long as several months.

Each test subject quantified his pain on the 0 to 10 pain intensity scale, as discussed in Example 1, before and after treatment. The lumbar and sacral radicular pain syndrom test results were as follows:

| Pain Syndrome Category | Number of Syndromes Treated | Average % Relief of Pain Reported |
|---|---|---|
| Degenerative disc disease | 195 | 84.7 % |
| Osteoarthritis | 57 | 87.5 % |
| Post-operative scarring | 40 | 78.6 % |
| Arthritis | 23 | 80.9 % |

## EXAMPLE NO. 3

In the preliminary uncontrolled tests discussed in Example No. 1, run on 396 subjects who each reported one or more pain syndromes, pain associated with arthritis of the knee was treated in 34 subjects. Two magnetic treatment devices, as shown in Fig. 1, were applied to each of the 34 subjects reporting knee pain. One treatment device 70 was placed over the joint space laterally and medial to the knee joint. Another treatment device (not shown) was placed on the opposite side of the knee one inch inferior and anterior to the proximal head of the fibula. The knee pain syndrome test results were as follows:

| Pain Syndrome Category | Number of Syndromes Treated | Average % Relief of Pain Reported |
|---|---|---|
| Arthritis - knee | 34 | 86.0 % |

## ONGOING STUDY

The preliminary uncontrolled study of treatment with the magnetic treatment device of the present invention shows promising results. The inventor is proceeding with a controlled study of the magnetic treatment device of the present invention in conjunction with the University of Alabama at Birmingham Medical Center and 4 other independent sites. The pain syndromes of Examples No. 1-3 above are each being studied in this controlled study.

The controlled study is a double blind crossover test in which each test subject will be tested with both a magnetic treatment device according to the present invention and a placebo device which cannot be distinguished from the magnetic treatment device of the present invention. The placebo device is identical to the magnetic treatment device of the present invention except that the metal in the device is not magnetized. The doctor applying the magnetic treatment device and the test subject will not know if a placebo device or the actual magnetic treatment device is being applied until after both the placebo and magnetic treatment device trials are completed on the specific test subject. The results of these tests will be submitted to the Patent Office when the tests are completed.

It will be apparent to those skilled in the art that modifications and variations can be made in the magnetic treatment device and the method for applying the magnetic treatment device of the present invention. The invention in its broader aspects, therefore is not limited to the specific details, representative methods and apparatus and illustrative examples shown and described above. Thus, it is intended that all matter contained in the foregoing description or shown in the accompanying drawings, shall be interpreted as illustrative and not in a limiting sense.

## Claims

1. A therapeutic permanent magnet device adapted for pain relieving placement on the bodies of living animals, comprising:

a plurality of magnet bodies having at least two positive and two negative magnetic poles substantially in a single plane, said magnetic poles being oriented to define the four vertices of a quadrilateral shape, said two positive poles defining opposite diagonal vertices and said two negative poles defining opposite diagonal vertices of the quadrilateral shape, each of said magnetic poles being magnetically attracted by said two oppositely charged poles and being magnetically repelled by said like charged pole; and

containment means (20) for holding said magnetic poles of said magnetic bodies in said orientation.

2. A therapeutic permanent magnet device in accordance with claim 1, further comprising attachment means (26) for fixing said containment means on the living body.

3. A permanent magnet device in accordance with claim 2, wherein said plurality of magnet bodies comprises four magnetic bodies (12, 14, 16, 18), each having at least one magnetically charged face, two (14, 18) of said magnetic bodies having a positive magnetic pole on one face, and the other two (12, 16) of said magnetic bodies having a negative magnetic pole on one face, said two positive and two negative magnetic poles on said magnetically charged faces of said four magnet bodies being in said quadrilateral orientation.

4. A permanent magnetic device in accordance with claim 3 wherein each of said four magnetic bodies (12, 14, 16, 18) includes two opposite faces (22, 24), one (22) of said two faces having a positive magnet pole thereon and the other (24) of said two faces having a negative magnetic pole thereon.

5. A permanent magnet device in accordance with claim 4 wherein each of said four magnetic bodies (12, 14, 16, 18) are substantially identical cylinders, each of said magnetic cylinders having two opposite end faces (22, 24), one of said two end faces having a positive magnetic pole thereon and the other of said two end faces having a negative magnetic pole thereon, the positive magnetic pole end faces (22) of two of said cylinders and the negative magnetic pole end faces of two of said cylinders being in a first plane, and the opposite negative magnetic pole end faces of two of said cylinders and the positive magnetic pole end faces of two of said cylinders being in a second plane.

6. A permanent magnet device in accordance with claim 5 wherein each of said magnetic cylinders comprises a neodymium magnet.

7. A permanent magnet device in accordance with claim 6 wherein each of said neodymium magnet cylinders has a diameter in the range of 6 mm to 13 mm.

8. A permanent magnet device in accordance with claim 7 wherein each of said magnetic cylinders exhibits an energy product of at least 25 MG-Oe.

9. A permanent magnet device in accordance with claim 5 wherein said quadrilateral shape is a parallelogram shape.

10. A permanent magnet device in accordance with claim 9 wherein said parallelogram shape is a rectangle shape.

11. A permanent magnet device in accordance with claim 10 wherein said rectangle shape is a square shape.

12. A permanent magnet device in accordance with claim 5 wherein said containment means (20) comprises a hypo-allergenic plastic, said four magnetic cylinders (12, 14, 16, 18) being embedded in said plastic.

13. A permanent magnet device in accordance with claim 5 wherein said containment (20) means comprises a woven sheath surrounding said four magnetic cylinders.

14. A permanent magnet device in accordance with claim 5 wherein said attachment means comprises a hypo-allergenic adhesive pad.

15. A permanent magnet device in accordance with claim 2 wherein said plurality of magnet bodies comprises two elongated magnet bodies (32, 34), each having a positive magnetic pole (35, 37) at one end and a negative magnetic pole (36, 38) at an opposite end, said two positive magnet poles and said two negative magnetic poles being in said quadrilateral orientation.

16. A therapeutic magnet device adapted for pain relieving placement on the human body, comprising: four magnet bodies (12, 14, 16, 18) each exhibiting an energy product of at least 25 MG-Oe, each of said magnet bodies having at least one magnetically charged face, two (14, 18) of said magnetic bodies having a positive magnetic pole on one face, the other two (12, 16) of said magnetic bodies having a negative magnetic pole on one face, said two positive poles of said two magnetic bodies (14, 18) and said two negative poles of the other two (12, 16) of said magnetic bodies being in a single plane, said two positive and two negative magnetic poles being oriented to define the four vertices of a rectangular shape, said two positive poles defining opposite diagonal vertices and said two negative poles defining opposite diagonal vertices of the rectangular shape;

containment means (20) for holding said two positive and two negative magnetic poles on the faces of said four magnetic bodies in said rectangular orientation; and ·

attachment means (26) for fixing said containment means on the human body.

17. A method of therapeutically placing a permanent magnet device on the human body to relieve pain, comprising the steps of:

assembling at least one group of magnet bodies having at least two positive magnetic poles substantially in a single plane, having at least two negative magnetic poles substantially in a single plane;

orienting said two positive poles and said two negative poles of said plurality of magnetic bodies in a single plane to define the four vertices of a rectangle shape with said two positive poles defining opposite diagonal vertices and said two negative poles defining opposite vertices of the rectangular shape;

fixing said orientation of said plurality of magnetic bodies in a single containment body;

selectively placing said containment body at a position on the skin of the human body over an area producing pain sensations; and attaching said containment body to the human body at said placement position.

18. A method in accordance with claim 17 further comprising the steps of repeating the steps of claim 17 for additional pluralities of magnet bodies in containment bodies for attachment to the human body at additional placement positions.

*FIG. 1*

*FIG. 2*

*FIG. 4*

*FIG. 3*

## FIG. 5

36    30    37

32    40

34

VI    VI

35

38

## FIG. 6

40    32    34

## FIG. 7

50    47

49    48

## FIG. 8

GAUSS READINGS
ONE-HALF INCH

Y-axis: GAUSS READING (THOUSANDS), from -1.6 to 1.4

X-axis: INCHES FROM CENTER, from 0.00 to 2.25

Legend: □ (-) RUQ    + (+) LUQ    ◇ (-) LLQ    △ (+) RLQ

EP 0 334 141 A1

## FIG. 9

( SURFACE )

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | -1 | -4 | -2 | +10 | +20 | +10 | +10 | 0 | 0 |
| 0 | 0 | -1 | -4 | -10 | +18 | -19 | +23 | +10 | +3 | +2 | 0 |
| 0 | 0 | -1 | -5 | +39 | N +1928 | S -1800 | -36 | +8 | +1 | 0 | 0 |
| 0 | 0 | +1 | +3 | -9 | -1988 S | +1900 N | +61 | -5 | -1 | 0 | 0 |
| 0 | +1 | +2 | +6 | +16 | -20 | -6 | -26 | -8 | -1 | 0 | 0 |
| 0 | +1 | +1 | +3 | +6 | +5 | -4 | -6 | -3 | 0 | 0 | 0 |
| 0 | 0 | 0 | +1 | +1 | 0 | -1 | -2 | -2 | -1 | 0 | 0 |
| 0 | 0 | 0 | 0 | +1 | +1 | -1 | -2 | -1 | -1 | 0 | 0 |
| 0 | 0 | 0 | 0 | 0 | 0 | 0 | 0 | 0 | 0 | 0 | 0 |

# FIG. 10

( .4 INCH ABOVE )

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | +1 | 0 |
| 0 | 0 | 0 | 0 | 0 | 0 | 0 | +1 | +2 | +1 | +1 | 0 |
| 0 | 0 | 0 | 0 | +2 | +3 | -1 | -1 | +1 | +1 | +1 | 0 |
| 0 | 0 | +1 | +2 | +12 | +33 | -33 | -18 | -1 | +1 | +1 | 0 |
| 0 | 0 | +1 | +2 | +43 | N +149 | S -118 | -82 | -2 | +1 | +1 | 0 |
| 0 | 0 | -1 | -3 | -19 | -133 S | +118 N | +98 | +4 | +1 | 0 | 0 |
| 0 | 0 | 0 | -1 | -22 | -196 | +28 | +33 | +2 | 0 | 0 | 0 |
| 0 | 0 | 0 | 0 | -2 | -8 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |

# FIG. 11

## (.8 INCH ABOVE)

| 0 | 0 | 0 | 0 | 0 | 0 | 0 | 0 | 0 | 0 | 0 | 0 |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | -1 | 0 | 0 | 0 | 0 |
| 0 | 0 | 0 | +1 | +3 | +2 | -2 | -4 | -2 | 0 | 0 | 0 |
| 0 | 0 | 0 | +2 | +10 | +17 | -11 | -19 | -4 | -1 | 0 | 0 |
| 0 | 0 | 0 | +3 | +16 | N +27 | S -18 | -17 | -3 | 0 | 0 | 0 |
| 0 | 0 | 0 | -2 | -6 | S -21 | +11 N | +14 | +3 | 0 | 0 | 0 |
| 0 | 0 | -1 | -3 | -14 | -23 | +6 | +13 | +3 | 0 | 0 | 0 |
| 0 | 0 | -1 | -2 | -5 | -6 | -1 | +2 | +1 | 0 | 0 | 0 |
| 0 | 0 | 0 | 0 | -1 | -2 | 0 | 0 | 0 | -1 | 0 | -1 |
| 0 | 0 | 0 | 0 | 0 | -1 | 0 | 0 | 0 | 0 | 0 | 0 |
| 0 | 0 | 0 | 0 | 0 | 0 | 0 | 0 | 0 | 0 | 0 | 0 |

# FIG. 12

(1.2INCHES ABOVE)

| 0 | 0 | 0 | 0 | 0 | 0 | 0 | 0 | 0 | 0 | 0 | 0 |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | -1 | 0 | 0 | 0 |
| 0 | 0 | 0 | +1 | +2 | +1 | -2 | -2 | -2 | 0 | 0 | 0 |
| 0 | 0 | 0 | +2 | +5 | +3 | -9 | -5 | -2 | -1 | 0 | 0 |
| 0 | 0 | 0 | +2 | +5 | N +4 | S -4 | -4 | -2 | 0 | 0 | 0 |
| 0 | 0 | 0 | -1 | -3 | S -2 | +1 N | +2 | 0 | 0 | 0 | 0 |
| 0 | 0 | -1 | -3 | -6 | -6 | +2 | +4 | +1 | 0 | 0 | 0 |
| 0 | 0 | -1 | -3 | -4 | -3 | 0 | +2 | +1 | 0 | 0 | 0 |
| 0 | 0 | 0 | -1 | -2 | -2 | 0 | 0 | 0 | 0 | 0 | 0 |
| 0 | 0 | 0 | 0 | 0 | -1 | 0 | 0 | 0 | 0 | 0 | 0 |
| 0 | 0 | 0 | 0 | 0 | 0 | 0 | 0 | 0 | 0 | 0 | 0 |

## FIG. 13

(1.6 INCHES ABOVE)

| | | | | | | | | | | | |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | -1 | 0 | 0 | 0 | 0 |
| 0 | 0 | 0 | 0 | +1 | +1 | 0 | -1 | -1 | 0 | 0 | 0 |
| 0 | 0 | 0 | +1 | +2 | +1 | -1 | -2 | -1 | -1 | 0 | 0 |
| 0 | 0 | 0 | 0 | +1 | N +1 | -1 S | -1 | -1 | 0 | 0 | 0 |
| 0 | 0 | 0 | 0 | -1 | S 0 | 0 N | 0 | 0 | 0 | 0 | 0 |
| 0 | 0 | -1 | -1 | -2 | -2 | 0 | +1 | 0 | 0 | 0 | 0 |
| 0 | 0 | 0 | -1 | -2 | -2 | 0 | 0 | 0 | 0 | 0 | 0 |
| 0 | 0 | 0 | 0 | -1 | -1 | 0 | 0 | 0 | 0 | 0 | 0 |
| 0 | 0 | 0 | 0 | -1 | 0 | 0 | 0 | 0 | 0 | 0 | 0 |
| 0 | 0 | 0 | 0 | 0 | 0 | 0 | 0 | 0 | 0 | 0 | 0 |

## FIG. 14

(2 INCHES ABOVE )

| 0 | 0 | 0 | 0 | 0 | 0 | 0 | 0 | 0 | 0 | 0 | 0 |
|---|---|---|---|---|---|---|---|---|---|---|---|
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | -1 | 0 | 0 | 0 | 0 |
| 0 | 0 | 0 | +1 | 0 | 0 | 0 | -1 | -1 | 0 | 0 | 0 |
| 0 | 0 | 0 | 0 | 0 | N 0 | S 0 | 0 | 0 | 0 | 0 | 0 |
| 0 | 0 | 0 | 0 | 0 | S -1 | 0 N | 0 | 0 | 0 | 0 | 0 |
| 0 | 0 | 0 | 0 | -1 | 0 | 0 | 0 | 0 | 0 | 0 | 0 |
| 0 | 0 | 0 | 0 | -1 | 0 | 0 | 0 | 0 | 0 | 0 | 0 |
| 0 | 0 | 0 | 0 | 0 | 0 | +1 | 0 | 0 | 0 | 0 | 0 |
| 0 | 0 | 0 | 0 | 0 | 0 | 0 | 0 | 0 | 0 | 0 | 0 |
| 0 | 0 | 0 | 0 | 0 | 0 | 0 | 0 | 0 | 0 | 0 | 0 |

## FIG. 15

(2.75 INCHES ABOVE)

FIG. 16

FIG. 17

FIG. 19

FIG. 18

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | FR-A-2 603 811 (MARUBENI) <br> * Page 5, lines 5-18; page 6, line 36 - page 7, line 4; page 15, lines 1-15; page 26, lines 20-29; page 27, line 1 - page 34, line 4; page 42, line 34 - page 43, line 2; page 48, line 32 - page 49, line 6; page 57, line 8 - page 59, line 3; page 67, lines 8-12; page 74, lines 28-31; page 76, lines 20-23 * <br> --- | 1-4,6,8 -18 | A 61 N    1/42 |
| X | EP-A-0 081 109 (ENERGY-PAK) <br> * Page 1, line 22 - page 2, line 17; page 5, lines 25-28 * <br> ----- | 1,2,5,7 ,11-14, 17,18 | |
| | | | TECHNICAL FIELDS SEARCHED (Int. Cl.4) <br><br> A 61 N |

The present search report has been drawn up for all claims

| Place of search | Date of completion of the search | Examiner |
|---|---|---|
| THE HAGUE | 06-06-1989 | LEMERCIER D.L.L. |